Europäisches Patentamt

European Patent Office

Office européen des brevets

(11) Publication number : **0 475 719 A2**

# (12) EUROPEAN PATENT APPLICATION

(21) Application number : **91308239.2**

(22) Date of filing : **10.09.91**

(51) Int. Cl.⁵ : **C07K 13/00, A61K 37/02, C12P 21/06**

(30) Priority : **11.09.90 JP 238944/90**

(43) Date of publication of application :
**18.03.92 Bulletin 92/12**

(84) Designated Contracting States :
**BE CH DE DK FR GB IT LI NL**

(71) Applicant : **Nakamura, Toshikazu**
**3-11-6 Midorigaoka**
**Higashi-ku, Fukuoka-shi 813 (JP)**

(72) Inventor : **Nakamura, Toshikazu**
**3-11-6 Midorigaoka**
**Higashi-ku, Fukuoka-shi 813 (JP)**

(74) Representative : **Arthur, Bryan Edward et al**
**Withers & Rogers 4 Dyer's Buildings Holborn**
**London EC1N 2JT (GB)**

(54) **Platelet derived growth regulating peptide.**

(57) Cytostatic agent PDGI-α is disclosed which lacks the transformation activity associated with TGF-β.

EP 0 475 719 A2

Jouve, 18, rue Saint-Denis, 75001 PARIS

# Figure 1

## Background to the Invention

This invention relates to a cytostatic agent (PDGI-$\alpha$) characterized by not having cancerous activity (transformation activity).

One factor used to control cell proliferation that is known in the prior art is the transforming growth factor-$\beta$ (hereinafter referred to as TGF-$\beta$). TGF-$\beta$ is a protein that was detected in cultured supernatants of cells during phenotypic transformation with MuSV (mouse RNA sarcoma virus) in 1978. It is a factor that accelerates NRK cell proliferation in soft agar (NRK originated in the kidneys of common rats).

TGF-$\beta$ has been named transforming growth factor because it is connected with phenotypic transformation and it was thought that the characteristics of this factor are present in specific tumorous cells. However, based on the results of research after the discovery of this agent, it is clear that TGF-$\beta$ is often present in specific blood platelets, and it is also evident that it is connected not only with transformation, but also with many physiological phenomena occurring inside living organisms.

The TGF-$\beta$ that was cultivated from human platelets was given the name TGF-$\beta$-1. So far there are 5 known types of agents related to TGF-$\beta$. These agents are found in humans, rats, pigs, and hens, and they were named $\beta$-1 through $\beta$-5.

TGF-$\beta$ is characterized by the same type of reactions in practically all the cells of a mammal. In addition, it can be also expected to have a healing effect on various types of diseases. The main in vitro functions of TGF-$\beta$ that have been determined so far include acceleration of the proliferation of certain cells (e.g., common cultured fibroblast cells and similar cells), suppression of the proliferation of epithelium cells and other interstitial cells, suppression of cell cultures, suppression of the proliferation of T-cells and other cells of the immune system, suppression of the proliferation of hematopoietic cells, etc. As far as confirmed activities of this agent in living organisms are concerned, it contributes to faster healing of wounds, to bone metabolism, to suppression of liver regeneration, etc.

Progress has also been made on the structure of TGF-$\beta$-1. Based on the present results of this research, TGF-$\beta$-1 is formed from precursors consisting of 390 amino acids (of the latent type). On the side of the carboxyl terminal it has a polypeptide with a 2 molecule dimer broken into 112 amino acids, and it can exist in activated state.

TGF-$\beta$ and its partial peptides can be produced by refining animal cells or by restructuring genes when revealed in cells. As far as clinical applications are concerned, TGF-$\beta$ has been used for instance in experiments for activation and acceleration of the proliferation of epithelium cells in connection with burns, as a therapeutic agent for healing of wounds after surgery, etc., as a therapeutic agent for eczema, as a therapeutic agent stimulating osseous growth for bone formation, for immunization suppression in diseases of the immune system, and in organ transplants for proliferation control of immune system cells, and for similar purposes.

However, utilization of TGF-$\beta$ brings about some major problems with respect to its clinical applications. Specifically, as the origin of its name suggests, TGF has the effect of promoting cancerous growth of normal cells. In other words, its big disadvantage is the fact that it activates transformation. Since there is a large danger of inducing cancer when TGF-$\beta$ is administered to a human patient, this fact represented a major obstacle to practical application of TGF-$\beta$.

## Summary of the Invention

Until now, a major obstacle preventing application of TGF-$\beta$ in clinical practice, which could have been expected because of a number of functions and effects such as those mentioned above, was the fact that it promotes cancerous development. Consequently, by eliminating this major defect of TGF-$\beta$ (i.e., its transformation activity), one can create an agent that has no cancerous effects, while it can be expected that this agent will maintain its various effects and functions that are suitable for clinical application. This would lead to development of an ideal agent when the agent is used as a drug.

Among the desired activities of the agent one can name:

(1) Activities contributing to acceleration of the proliferation of certain cells (e.g., cultured fibroblast cells and similar cells), suppression of the proliferation of epithelium cells and other interstitial cells, suppression of cell cultures, suppression of the proliferation of T-cells and other cells of the immune system, suppression of the proliferation of hematopoietic cells, etc.

(2) Acceleration of the healing of wounds in vivo, stimulation of osseous growth for bone formation, suppression of liver regeneration, and similar activities.

(3) In addition, it is also desirable for the agent not to have transformation activities (represented by inducing colony formation in NRK-49F cells proliferating in soft agar).

Thus one aim of the present invention was to manufacture a biologically active substance that will fulfill some or all of the criteria named above.

The agent of the present invention is characterized by the following:

(1) PDGI-α (Platelet Derived Growth Inhibitor-alpha), originating in human blood platelets, has these properties:

(a) Adsorption in S-Sephalose under conditions of pH 8 without adsorption in Biogel P60 in aqueous solution of 1N acetic acid.

(b) Inactivation of approximately 50% when processed for 3 minutes at 100°C, inactivation of 100% with 0.065 dithiothreitol, although the substance is stable in 1N acetic acid.

(c) Molecular weight was measured by SDS-polyacrylamide gel electrophoresis. A unit band of approximately 26 kD occurs under conditions of non-reduction. Unit bands of approximately 13 kD, 8 kD, 5 kD bands were detected under conditions of reduction.

(d) In negative phase gel column, soluble in approximately 37 - 39% fractions using the inclined dissolution method with an aqueous solution of 35 - 50% acetonitrile.

(e) Although PDGI-α accelerates proliferation of common cultured fibroblast cells and suppresses proliferation of epithelium cells and other interstitial cells, no activation of NRK-49F cell colony formation in soft agar was displayed.

(2) PDGI-α has N-terminal amino acid sequences described below; in addition, it is also characterized by consisting of three subunits whose molecular weights are described below (according to SDS-polyacrylamide gel electrophoresis under conditions of reduction):

subunit a-ALDTNYCFSSTE, approximately 13 kD;

subunit b-ALDTNYCFSSTE, approximately 8 kD; and

subunit c-NQHNPGASAAPC, approximately 5 kD.

(3) PDGI-α, with S-S linkage, consists of three subunits having amino acid sequences described below:

```
a:    A L D T N Y C F S S      T E K N C C V R Q L

      Y I D F R K D L G W      K W I H E P K G Y H

      A N F C L G P C P Y      I W S L D T Q Y S K

      V L A L Y N Q H N P      G A S A A P C C V P

      Q A L E P L P I V Y      Y V G R L P K V E Q

      L S N M I V R S C K      C S


b:    A L D T N Y C F S S      T E K N C C V R Q L

      Y I D F R K D L G W      K W I H E P K G Y H

      A N F C L G P C P Y      I W S L D T Q Y S K

      V L A L Y

c:    N Q H N P G A S A A      P C C V P Q A L E P

      L P I V Y Y V G R L      P K V E Q L S N M I

      V R S C K C S
```

## Brief Description of the Drawings

Figure 1 shows elution patterns of proteins in Sephacryl S-300 gel column refining. The continuous full line shows absorbance at 492 nm of Example 1 (1). In addition, small solid circles show the amounts of synthetic DNA.

Figure 2 shows elution patterns of proteins in S-Sephacryl refining of Example 1. The continuous full line shows absorbance at 492 nm, while small solid circles show the amounts of synthetic DNA. The dashed line shows the concentration of NaCl in the elution solvent.

Figure 3 shows elution patterns of proteins in negative phase gel column refining of Example 1 (1). The continuous full line shows absorbance at 492 nm, while the broken line displays the concentration of acetonitrile in the elution solvent.

Figure 4 shows SDS-polyacrylamide gel electrophoresis patterns ot PDGI-α and TGF-β under non-reduction conditions. It relates to Example 1 (1).

Figure 5 shows the SDS-polyacrylamide gel electrophoresis patterns of PDGI-α and TGF-β under reduction conditions. It relates to Example 2.

Figure 6 shows the effect of PDGI-α and TGF-β on inducing colony formation of NRK-49F cells in soft agar and the effect of suppression of proliferation (full line with small solid circles) in liver cells. It relates to Example 4. Although PDGI-α has the effect of suppressing proliferation control in liver cells, no effect on inducing colony growth of NRK-49F cells in soft agar was displayed.

Figure 7 is a photo taken during microscopic observation of the effect of PDGI-α and TGF-β on inducing colony growth of NRK-49F cells in soft agar. It relates to Example 4. Figure (1) represents a control (addition of 2 ng/ml of EGF (growth factor for epithelium cells)); Figure (2) represents addition of 2 ng/ml TGF-β-1 and of 2 ng/ml of EGF; and Figure (3) represents addition of 3 ng/ml of PDGI-α together with 2 ng/ml of EGF. Although colony formations with a diameter of at least 50 μ were registered in figure (2), no formations were displayed in figure (3).

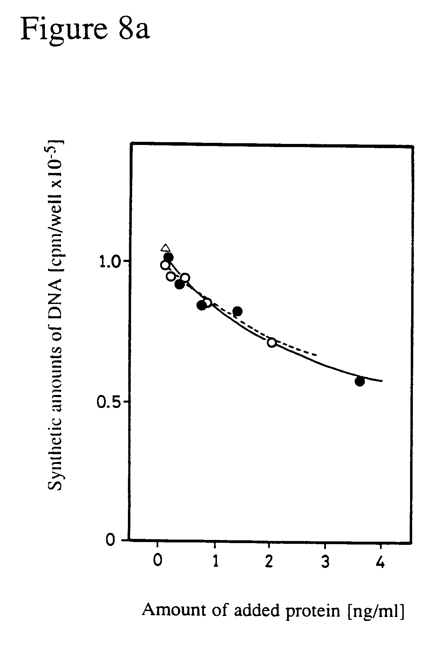

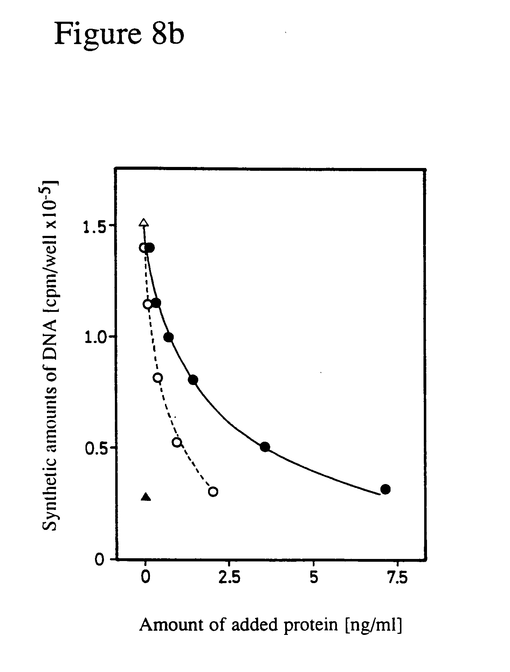

Figure 8 shows the effect of PDGI-α and TGF-β on the suppression of proliferation in A 431 cells (see Figure 8-a) and in BRL-3-A cells (see figure 8-b). The figures relate to Example 5. They show the effect of suppression of proliferation of both PDGI-α (small solid circles and full line) and of TGF-β (small hollow circles and broken line). When only 10% bovine fetal serum was added for control (indicated by hollow triangle), proliferation was not suppressed (see figure 8-a). On the other hand, when 10% bovine fetal serum was not added (indicated by full triangle), no proliferation was displayed (see figure 8-b).

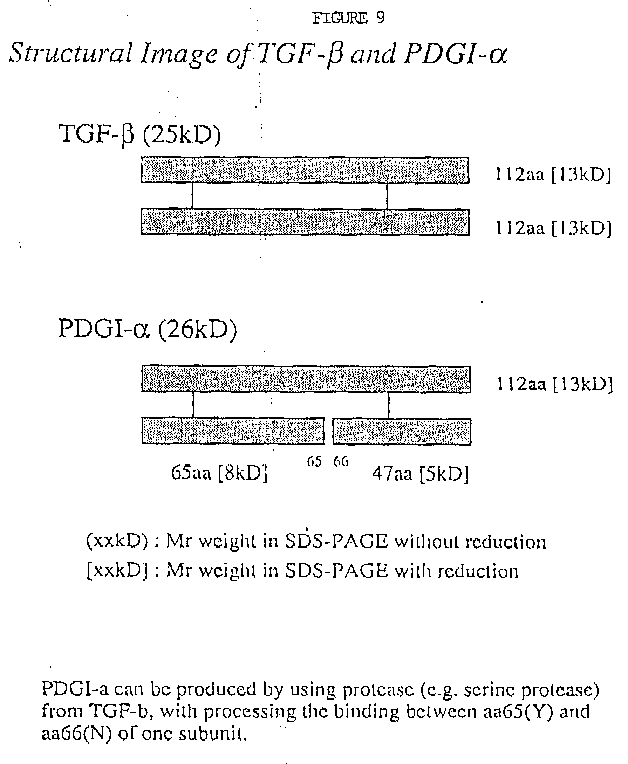

Figure 9 shows the structural relationship of TGF-β and PDGI-α.

Detailed Description of the Invention

As far as the polypeptides of this invention are concerned, they are described by the following amino acids: A is alanine, C is cysteine, D is aspartic acid, E is glutamic acid, F is phenyl alanine, G is glycine, H is histidine, I is isoleucine; K is lysine, L is leucine, M is methionine, N is asparagine, P is proline, Q is glutamine, R is arginine, S is serine, T is threonine, V is valine, W is tryptophane and Y is tyrosine.

The following is a detailed description of the procedures used:

First of all, the present invention is based on the discovery of biologically active polypeptides representing an accelerating factor of in vitro proliferation of primary cultures of liver cells of adult rats originating in the animal cell structure. The proliferation factor for growth of mature liver cells was given the name Hepatocyte Growth Factor or HGF (Biochem. Biophys. Res. Commun., volume 122, page 1450, 1984), and its structure was successfully explained (human HGF: Nature, volume 342, page 440, 1989).

Next, during the course of research on HGF, it was discovered that certain types of peptides, similar to HGF, are present in human blood platelets. These peptides, unlike HGF, strongly suppress proliferation of cultured liver cells, and one of these peptides is called TGF-β.

During the course of further intensive research a substance with a property that is totally different from TGF-β was discovered. This substance also displays the effect of suppressing proliferation of cultured liver cells. Although its structure is similar to that of TGF-β, this substance does not display any transformation activity (that is to say it has no activity inducing colony formation of NRK-49F cells in soft agar). In addition, its structure has been determined, and since it was successfully obtained as protein, this made it possible to complete this invention.

PDGIα can be obtained as explained below:

It can be obtained for instance by using an enzyme which breaks in a specific manner the peptide link on the carbonyl side of tyrosine. For instance, in the transforming growth factor TGF-β (originating in human blood platelets), the reaction of chymotrypsin or pepsin at a low temperature (about 0 - 2° C) for about 1 week can be used.

In addition, it is also possible to conduct extraction refining of PDGIα from human blood platelets according to the method of Example 1. Specifically, it is possible to conduct centrifugal separation after ultrasonic crushing of human blood platelets, and after refining with Sephacryl S-300, Biogel P-60, and S-Sephalose gel columns, it is possible to obtain a refined product by high speed liquid chromatography of C4 negative phase silica gel.

Furthermore, PDGIα comprises subunits a, b, and c, and since the subunits b and c are consistent with

5

the sequences that are broken between the 65th and 66th sequence of the polypeptide of subunit a, it is possible to integrate genes having a base sequence coding for polypeptides of subunit a, and after dimer formation, it is possible to use a suitable restriction enzyme to break the linkage.

To be even more specific, it is possible to produce each subunit with common genetic engineering methods, which are well known in the art, such as the methods described below. Specifically:

(a) Human tissue cells and blood, and RNA extracted from blood plasma, is used as the template to form a cDNA combination, and plasmid pBR 322, originating in the large intestine, or bacteriophage lambda gt 11 or a similar recombination pattern, is used.

(b) The cDNA library is formed by integration of Escherichia coli (e.g., E. coli NM514) or of similar host cells.

(c) Using a partial oligonucleotide $^{32}$P or a similar radioactive element based on the base sequence coding for the amino acid sequence of PDGI-$\alpha$ as a probe label, one can select the desired cDNA by utilizing the colony hybridization method, the plaque hybridization method, or a similar method known in the art.

(d) The cDNA sequence is determined by the Maxum and Gilbert chemical method (Proc. Natl. Acad. Sci, volume 74, page 560, 1970) or by a similar method known in the art.

(e) The cDNA is broken by restriction enzyme from the vector containing the cDNA coding for all the amino acid sequences of the subunits of PDGI-$\alpha$.

(f) Integration is done using DNA ligase with restriction enzyme, with an appropriate onset vector (e.g., plasmid bPR 322 originating in E. coli or neoplasm virus SV 40, etc.).

(g) Onset can be obtained by introduction into C 127 cells of mice, COS cells of monkeys, etc.

Next, the three subunits of PDGI$\alpha$ (a, b, and c) are linked chemically or enzymatically to obtain PDGI-$\alpha$.

It is also possible to link only the subunit a chemically or enzymatically to create a dimer, and then to break the bond between 65th tyrosine and 66th asparagine (from the N-terminus on one subunit side) by use of serine protease or of a similar suitable protease in order to obtain PDGI-$\alpha$.

The term "PDGI-$\alpha$" is meant to include both platelet-derived and recombinant materials (obtained by recombinant DNA technology known in the art), preferably primate origin; most preferably the primate is a human.

Examples

Example 1: (1) Isolation-Retining of PDGI-$\alpha$ from Human Blood Platelets.

Isolation-refining was conducted with human blood platelets according to the method described below, using PDGI-$\alpha$ of this invention.

2,000 samples of human blood platelet concentrated solution was centrifuged at 300 x g for 5 minutes and red blood cells were eliminated. The supernatant was recovered and centrifuged at 10,000 x g for 15 minutes. The resulting sediment was twice processed by centrifugal washing in approximately 8,000 ml of iced phosphate buffered saline (hereinafter referred to as "PBS"), and after that it was suspended in approximately 2,500 ml of PBS (10 mM EDTA, pH 7.4), and centrifuged at 25,000 x g for 40 minutes.

The resulting sediment was suspended in approximately 5,000 ml of iced PBS (10 mM EDTA, pH 7.4), and while being cooled, it was treated with ultrasonic waves (Insonator model 200 M, made by Kubota Company); after the crushing was completed, it was centrifuged 105,000 x g for 60 minutes and the supernatant was filtered using an ultrafiltration membrane (Minitan, cut off 10,000, made by the Milipore Company), and a concentration of about 10 fold was obtained. After dialysis in PBS (6 M urea, pH 7.4), which lasted 2 days, urea was adjusted to 8 M, and the supernatant was centrifuged at 105,000 x g for 30 minutes.

Refining was conducted according to the method described under (2), which indicated cytostatic activity.

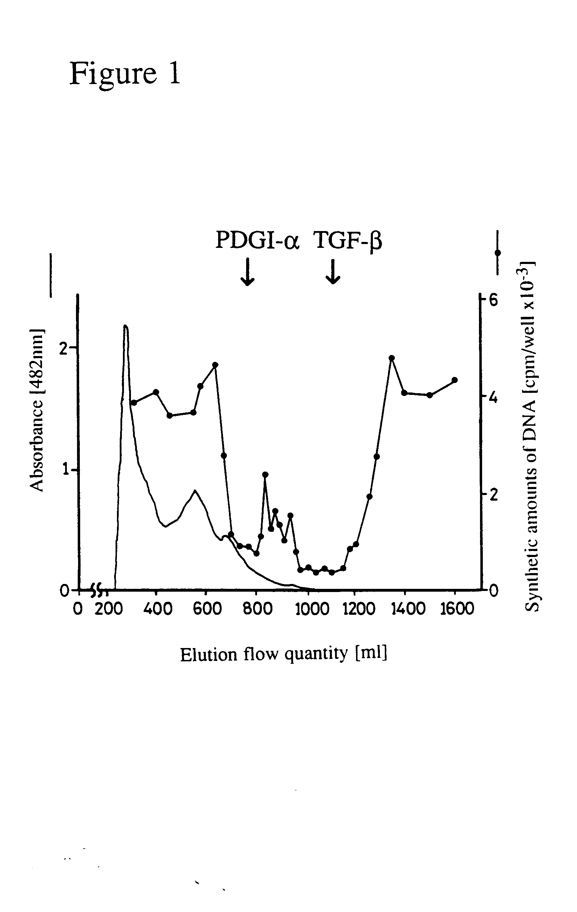

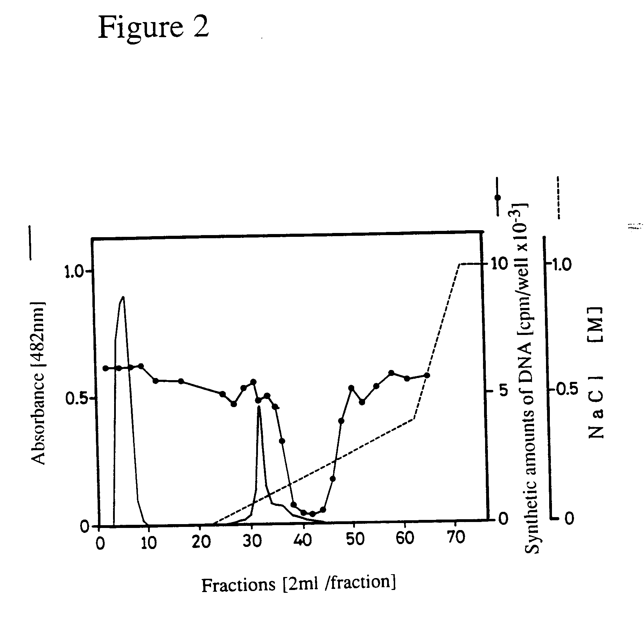

The samples described were refined using Sephacryl S-300 (made by the Pharmacia Company) in a gel column (see Figure 1). Dissolved active fractions were concentrated using an ultrafiltration membrane. After dialysis, which was conducted in an aqueous solution of 1 N acetic acid, separation refining was done with Biogel P-60 in a gel column. After the non-absorption fractions were dried by freeze drying, the freeze dried fractions were dissolved in an aqueous solution of 1 N acetic acid, and dialysis was conducted in 25 mM Tris-HCl buffer solution (pH 8.0), containing 6 M urea. After the sample was added to an S-Sephalose gel column (manufactured by the Pharmacia Company), non-absorption components were washed in the sane buffer solution, and after that adsorption fractions were dissolved in a linear gradient of aqueous solution of 0 --> 1 M NaCl (see Figure 2). In addition, TGF-$\beta$-1 was isolated and refined from non-absorption components. The example of this invention described below used this TGF-$\beta$-1 as the TGF-$\beta$ sample.

After the obtained PDGI-$\alpha$ fractions were concentrated using a diaflow membrane (cut off 10,000, manufactured by the Amicon Company), sinking was conducted in YMC C$_4$HPLC column with an aqueous solution of 0.1% trifluoroacetic acid, and also the non-absorption components were washed using the same solvent.

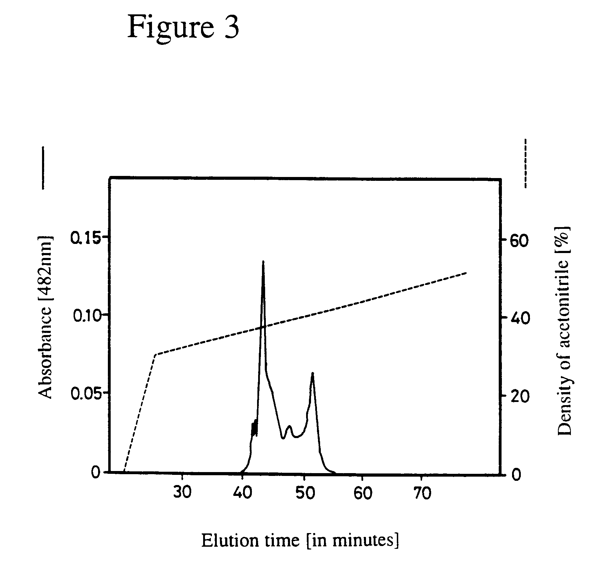

After dissolution with 0 --> 90% of a linear gradient of acetonitrile aqueous solution, acetonitrile was removed from the PDGI-$\alpha$ fractions using decompression. Sinking was conducted in YMC $C_4$HPLC column with an aqueous solution of 0.1% heptafluoroacetic acid (C4 negative phase). After washing the non-absorption components in the same solvent, extraction was conducted with a linear gradient of 35 - 50% acetonitrile aqueous solution and refined PDGI-$\alpha$ was obtained in 37 - 39% fractions (see Figure 3).

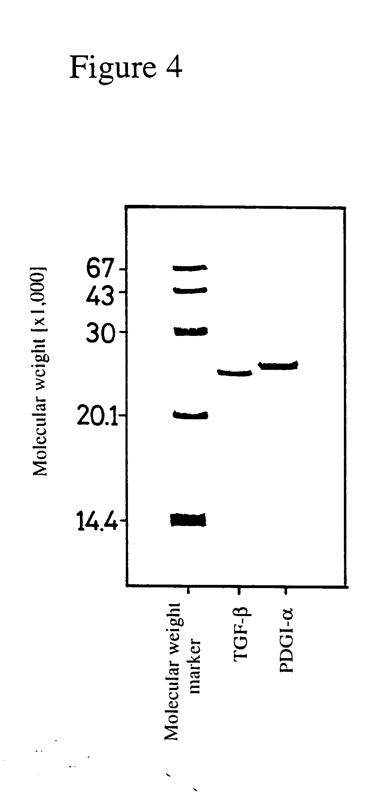

Refined PDGI-$\alpha$ and TGF-$\beta$ obtained in this example were separated with SDS-polyacrylamide gel electrophoresis. Figure 4 shows the results, which had a silver coloring. The molecular weight of PDGI-$\alpha$ was approximately 26 kD, and the molecular weight of TGF-$\beta$ was approximately 25 kD.

Example 1: (2) Cytostatic Effect on Primary Culture of Adult Rat Liver Cells

The cytostatic effect of PDGI-$\alpha$ was assessed using a primary culture of adult rat liver cells.

Separation and refining of hepatocytes of Wistar rats was conducted using the collagen reflux method. The hepatocytes obtained were suspended in Williams E culture medium that included 5% calf blood serum with a density of $2.5 \times 10^5$ (units)/ml; 0.5 ml/well was placed in 24 well multi-plates. Under the conditions of 5% $CO_2$, 30% $O_2$, and 65% $N_2$, after cultivation at 37° C lasting 20 hours, the desired amount of the sample was dissolved in 50 $\mu$l of PBS, containing 2.5 mg/ml of bovine serum albumin together with replacement with serum-free Williams E culture, containing $1 \times 10^{-7}$ M insulin (manufactured by the Sigma Company), and 10 ng/ml EGF (recombinant human EGF, Earth Chemical Company).

Atter culturing for 12 hours, 10$\mu$l of $^3$H-deoxythymidine (25 $\mu$Ci/ml) was added to each well, and 15 minutes before adding $^3$H-deoxycytimidine to the control group, 5 $\mu$l/ml of aphidicolin was added. After culturing lasting 24 hours, a tritiated label was formed, the cells were washed twice in a PBS solution with pH of 7.4, and they were retained in an aqueous cooling solution of 10% trichloro acetate. Solubilization of the cells was done with 0.5 ml per well of an aqueous solution of 1 N sodium hydroxide, and their radioactivity was measured using a gamma counter.

After measuring radioactivity, a part of the sample was removed and its protein amount was measured according to the Lowry method. When the test samples were added, the difference of the tritium amount incorporated into hepatocytes was determined from the control count, and this difference was calculated per 1mg of rat hepatocyte protein, and DNA synthesis activity was determined (cpm/mg protein).

Example 2 - Determination of the Amino Acid Sequence of the N-terminus of PDGI-$\alpha$.

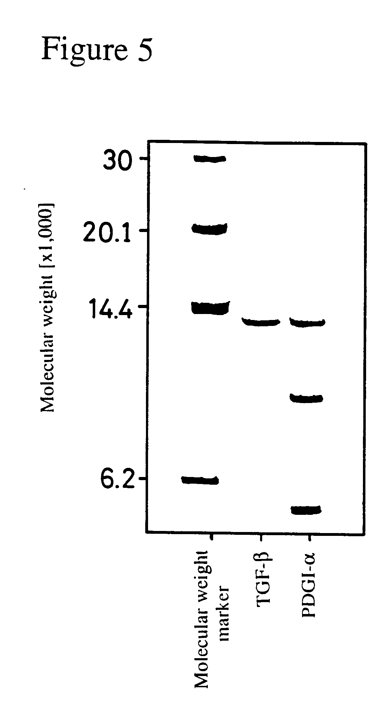

Refined PDGI-$\alpha$ obtained in Example 1 and TGF-$\beta$ (obtained in the refining process of PDGI-$\alpha$ of Example 1) were dissolved in an aqueous solution of 2-mercaptoethanol, and after reduction was achieved with heat treatment, separation was conducted using SDS-polyacrylamide gel electrophoresis. As shown in Figure 5, TGF-$\beta$ displayed a single band of approximately 13 kD, while with PDGI-$\alpha$ there were three bands of approximately 13 kD, 8 kD, and 5 kD. Thus TGF-$\beta$ consisted of two subunits with the same molecular weight, while PDGI-$\alpha$ clearly consisted of three subunits with differing molecular weights (hereinafter, these subunits are called a (approximately 13 kD), b (approximately 8 kD), and c (approximately 5 kD)). Parts corresponding to each band separated from PDGI-$\alpha$ were sectioned, extracted with PBS (pH 7.4), and the N-terminus amino acid sequence was determined using a peptide sequencer (type 477 A, manufactured by the Applied Biosystems Company). The results were:

a: ALDTNYCFSSTE;
b: ALDTNYCFSSTE; and
c: NQHNPGASAAPC.

Example 3

All the amino acid sequences of PDGI-$\alpha$ were determined using the following method.

Refined PDGI-$\alpha$ obtained in Example 1 was dissolved in an aqueous solution of 2-mercaptoethanol, and after heat treatment, reduction of cysteine linkages was achieved. In order to drastically lower the peptide chains of each subunit, the N-terminus was treated with methyl sulfone using methane sulfonyl chloride. Sinking was conducted in YMC $C_4$HPLC column with an aqueous solution of 0.1% trifluoroacetic acid, and separation of the three subunits was conducted with a linear gradient of 0 --> 50% acetonitrile aqueous solution. Each subunit obtained in this manner was treated by chymotrypsin, and separation was then conducted one more time with HPLC. The resulting 10 to 30 amino acid residues of peptide chain fragments were determined as N-terminus amino acid sequences using a peptide sequencer. As a result, the following amino acid sequences of the three subunits were determined:

```
a:   A L D T N Y C F S S        T E K N C C V R Q L

     Y I D F R K D L G W        K W I H E P K G Y H

     A N F C L G P C P Y        I W S L D T Q Y S K

     V L A L Y N Q H N P        G A S A A P C C V P

     Q A L E P L P I V Y        Y V G R L P K V E Q

     L S N M I V R S C K        C S


b:   A L D T N Y C F S S        T E K N C C V R Q L

     Y I D F R K D L G W        K W I H E P K G Y H

     A N F C L G P C P Y        I W S L D T Q Y S K

     V L A L Y


c:   N Q H N P G A S A A        P C C V P Q A L E P

     L P I V Y Y V G R L        P K V E Q L S N M I

     V R S C K C S
```

Example 4

One purpose of this example was to compare TGF-β and PDGI-α with respect to the accelerating effect on colony formation of NRK-49F cells in soft agar and to the effect on proliferation control in rat liver cells.

Using the refined product obtained in Example 1, the effect of the operation of PDGI-α was compared to TGF-β.

As far as the cell proliferation suppressing activity is concerned, we were able to confirm it with proliferation suppressing effect on primary culture of adult rat liver cells according to the method of Example 1, described under item (2).

With respect to transformation activity, using the method described below, we were able to confirm the existence or nonexistence of the accelerating effect on colony formation of NRK-49F cells in soft agar.

NRK-49F cells were gathered from Dulbecco-Eagle culture medium (manufactured by the Flow Laboratory Company, U.S., hereinafter referred to as DME), which included 10% bovine fetal serum. After culturing and treatment with trypsin, the cells were gathered using centrifugal force. Agar media with 0.3% of DME, which included 10% bovine fetal serum, was kept at 40° C, and after suspension so as to increase the density of NRK cells to 3,000/ml, PDGI-α and TGF-β was added to epithelial cell growth factor EGF (originating in mouse glands) corresponding to 2 ng/ml, and dissolved in a specified amount of PBS. Then 0.6% of agar culture media, which included 10% bovine fetal serum, was added on a plate with a diameter of 35 mm in doses of 2 ml. After cooling and solidifying, NRK cells were suspended and 0.3% of agar culture media was added in doses of 1 ml. After solidification was induced at room temperature, culturing was conducted for 10 days at 37° C and under the following conditions: 10% of $CO_2$, 25% of $O_2$, and 65% of $N_2$. After the culturing was finished, PBS containing 10% formalin was added for solidification, and the number of colonies with a diameter of at least 50 μm was counted using microscopic observation.

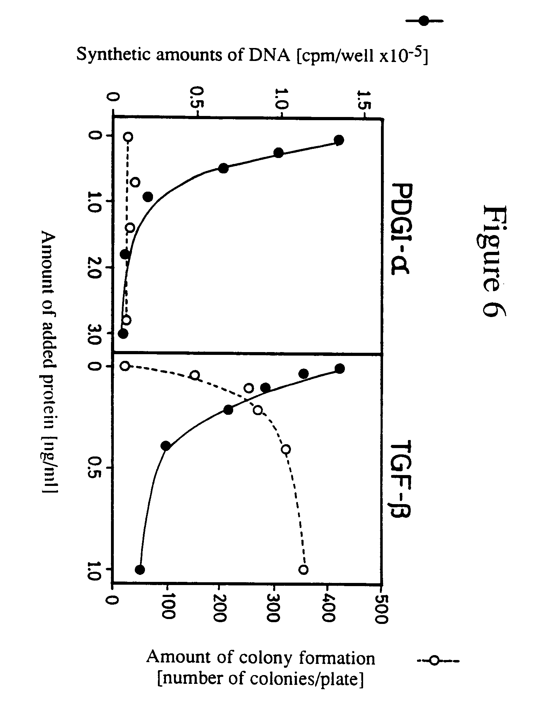

Figure 6 shows the results. There was almost no difference that we were able to observe between both substances as far as the effect of the proliferation suppressing agents on the liver cells and as far as the effect of the dose dependency is concerned. On the other hand, we were able to confirm that there was a great difference between the two substances as far as the colony formation of NRK cells is concerned. Specifically, TGF-β clearly had the effect of accelerating colony formation, and its dose dependency was consistent with the increase of the effect on the suppression of proliferation of liver cells, while PDGI-α did not accelerate colony growth (even when double the concentration achieved during the peaks of suppression of liver cell proliferation was added, colony formation was not induced).

Based on these results, although the PDGI-α of this invention has the same effect as TGF-β with respect to the effect on suppression of proliferation in liver cells, it was clearly and unexpectedly proved that it does not have transformation activity.

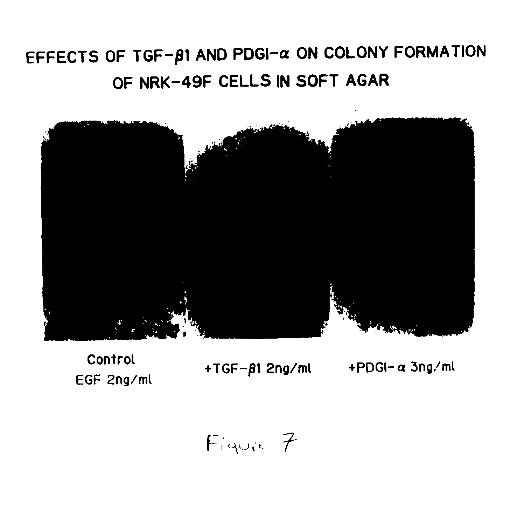

Figure 7 shows the results of microscopic observation of the status of the colony formation after the experiment with the NRK-49F cells of this example of this invention.

Example 5

This example was conducted to compare the effect of TGF-β and PDGI-α on the suppression of proliferation of epithelial cells.

The effect on suppression of proliferation of cells by PDGI-α was examined with established cell lines of epithelial cells (A431 and BRL-3A). The A431 and BRL-3a cells were obtained from the cell bank of the Foundation for Promotion of Cancer Research (JCRB). Each cell culture was precultured in DME culture containing 10% bovine fetal serum, and centrifugal force was used to gather the cella. Each cell culture was suspended in the same culture medium at $1.0 \times 10^5$/ml, and 0.5 ml of each culture was added to each well. After culturing for 24 hours, a specified amount of PDGI-α and TGF-β was added and the product was transferred to the same culture medium. After 20 hours of culturing, 0.5 μCi $^{125}$I-deoxyuridine was added to each well. Then, after 4 hours of culturing, incorporation into the cells was induced ($^{125}$I), and the product was washed twice with PBS with a pH of 7.4. After solidification with a cooling solution of 10% trichloro acetate, solubilization of the cells was done with 0.5 ml per well of an aqueous solution of 1 N sodium hydroxide, and their radioactivity was measured using a gamma counter. The results were shown as a count per 1 well (cpm/well).

As shown in Figure 8, which indicates the effect on suppression of proliferation in A431 cells (see Figure 8-a), absolutely no difference could be observed between PDGI-α and TGF-β. Although there was a slight difference related to the effect of concentration on BRL-3A cells (see Figure 8-b), the same type of affect on suppression of proliferation was displayed.

Thus PDGI-α has the effect of suppressing proliferation of liver cells which is identical to that of TGF-β. On the other hand, in contrast to TGF-β, PDGI-α is also characterized by not having cancerous activity (transformation activity), which is a very important effect. That is why it represents a safe cytostatic agent since it has no side effects conducive to growth of cancerous cells, and thus it can be expected to be applicable as a therapeutic agent with eczema, as a therapeutic agent stimulating osseous growth for bone formation, for immunization suppression in immunization diseases and for similar purposes. An effective amount of the cytostatic agent would be utilized. An effective amount would be an amount to effectively treat the medical condition being treated (e.g., an amount effective to treat eczema). The cytostatic agent may be used as a pharmaceutical composition.

The pharmaceutical composition of this invention may contain the active compounds (e.g., PDGI-α) together with a solid or liquid pharmaceutically acceptable nontoxic carrier. such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solution and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, magnesium carbonate, magnesium stearate, sodium stearate, glycerol monoatearate, talc, sodium chloride, dried skim milk, glycerol, propylene glycol, water, ethanol and the like. These compositions can take the form of solutions, suspensions, tablets, pills, capsules, powders, sustained-release formulations and the like. Suitable pharmaceutical carriers are described in "Remington's Pharmaceutical Sciences" by E. W. Martin. Such compositions will contain an effective therapeutic amount of the active compound together with a suitable amount of carrier so as to provide the form for proper administration to the host. While intravenous injection is a very effective form of administration, other modes can be employed. The active compound can also be combined with known emollient bases to form a cream for external use (e.g., for the treatment of eczema). The active compound can also be suspended in biocompatible gel.

U.S. Patent No. 5,035,887; 5,034,375; and 4,983,581 are incorporated by reference in their entirety.

Further variations and modifications of the invention will become apparent to those skilled in the art from the foregoing and are intended to be encompassed by the claims appended hereto.

The Japanese Priority Application 238944/90, filed on September 11, 1990, is relied on and incorporated by reference.

Explanation of Figures

Figure 1:

1. Absorbance (482 nm)
2. Elution flow quantity (ml)
3. Synthetic amounts of DNA (cpm/well X $10^{-3}$)

Figure 2:

1. Absorbance (482 nm)
2. Fractions (2 ml/fraction)
3. Synthetic amounts of DNA (cpm/well X $10^{-3}$)

Figure 3:

1. Absorbance (482 nm)
2. Elution time (in minutes)
3. Density of acetonitrile (%)

Figure 4:

1. Molecular weight (x 1000)
2. Molecular weight markers

Figure 5:

1. Molecular weight (x 1000)
2. Molecular weight markers

Figure 6:

1. Synthetic amounts of DNA (cpm/well x $10^{-5}$)
2. Amount of added protein (ng/ml)
3. Amount of colony formation (number of colonies/plate)

Figure 8a:

1. Synthetic amounts of DNA (cpm/well x $10^{-5}$)
2. Amount of added protein (ng/ml)

Figure 8b:

1. Synthetic amounts of DNA (cpm/well x $10^{-4}$)
2. Amount of added protein [ng/ml]


**Claims**

1. PDGI-$\alpha$ comprising subunits a, b and c.

2. The PDGI-$\alpha$ according to claim 1,
   (a) wherein subunit a has the following amino acid sequence:

```
A L D T N Y C F S S          T E K N C C V R Q L

Y I D F R K D L G W          K W I H E P K G Y H

A N F C L G P C P Y          I W S L D T Q Y S K

V L A L Y N Q H N P          G A S A A P C C V P

Q A L E P L P I V Y          Y V G R L P K V E Q

L S N M I V R S C K          C S;
```

(b) wherein subunit b has the following amino acid sequence:

```
b.  A L D T N Y C F S S       T E K N C C V R Q L

    Y I D F R K D L G W       K W I H E P K G Y H

    A N F C L G P C P Y       I W S L D T Q Y S K

    V L A L Y;  and
```

(c) wherein subunit c has the following amino acid sequence:

```
    N Q H N P G A S A A       P C C V P Q A L E P

    L P I V Y Y V G R L       P K V E Q L S N M I

    V R S C K C S.
```

3. The PDGI-$\alpha$ according to claim 1, wherein subunit b is bonded to subunit a and subunit c is bonded to subunit a.

4. A pharmaceutical compsition comprising PDGI-$\alpha$ according to claim 1 and a pharmaceutically acceptable carrier.

5. The pharmaceutical composition according to claim 4, wherein said PDGI-$\alpha$ is present in an effective amount.

6. A method of making PDGI-$\alpha$ according to claim 1, comprising reacting TGF-$\alpha$ with chymotrypsin or pepsin at about 0 - 2° C for about 1 week.

7. A method of treating a patient without causing transformation activity or promoting cancerous growth of cells in said patient, comprising administering to said patient an effective amount of the pharmaceutical composition according to claim 4.

# Figure 1

# Figure 2

# Figure 3

Elution time [in minutes]

# Figure 4

# Figure 5

# Figure 6

EFFECTS OF TGF-$\beta$1 AND PDGI-$\alpha$ ON COLONY FORMATION
OF NRK-49F CELLS IN SOFT AGAR

| Control | +TGF-$\beta$1 2ng/ml | +PDGI-$\alpha$ 3ng./ml |
| EGF 2ng/ml | | |

Figure 7

EP 0 475 719 A2

# Figure 8a

Figure 8b

## FIGURE 9

## *Structural Image of TGF-β and PDGI-α*

### TGF-β (25kD)

112aa [13kD]

112aa [13kD]

### PDGI-α (26kD)

112aa [13kD]

65aa [8kD]    65  66    47aa [5kD]

(xxkD) : Mr weight in SDS-PAGE without reduction

[xxkD] : Mr weight in SDS-PAGE with reduction

PDGI-a can be produced by using protease (e.g. serine protease) from TGF-b, with processing the binding between aa65(Y) and aa66(N) of one subunit.